# EUROPEAN PATENT SPECIFICATION

(11) **EP 2 744 833 B1**
(45) Date of publication and mention of the grant of the patent: **28.09.2016**
(21) Application number: 12753234.9
(22) Date of filing: 20.08.2012
(51) Int. Cl.: C08F 2/40, C07C 7/20, C08F 12/08, C08F 20/04

(54) **INHIBITOR COMPOSITIONS AND METHODS OF USE**
INHIBITORZUSAMMENSETZUNGEN UND VERWENDUNGSVERFAHREN DAFÜR
COMPOSITIONS INHIBITRICES ET PROCÉDÉS D'UTILISATION

(30) Priority: 19.08.2011 GB 201114332
(43) Date of publication of application: 25.06.2014
(73) Proprietor: Nufarm UK Limited, Bradford, West Yorkshire BD12 9EJ (GB)
(72) Inventor: PRICE, Peter, Ilkley Yorkshire LS29 6LA (GB); DUCHAMP, Guillaume, F-92230 Gennevilliers (FR); GLOSSOP, Angela Jane, Rotherham South Yorkshire S66 9NR (GB); FAWBERT, Andrew Nigel, Brighouse Yorkshire HD6 2BH (GB); DWYER, Stephanie Mary, Oxenhope Yorkshire BD22 9NP (GB)
(74) Representative: Woollaston, Daniel John
(86) International application number: PCT/GB2012/052031
(87) International publication number: WO 2013/027042

(56) References cited:
- CN-A- 101 838 058
- US-A- 5 707 551
- US-A- 5 906 962
- US-A1- 2007 293 549

## Description

### Field of the Invention

The present invention relates to composition for use in the production and processing of ethylenically unsaturated monomers for effectively preventing unwanted polymerisation reactions during said processes. Specifically, the invention is concerned with a formulation and method for the supply of active polymerisation inhibitor compounds of low solubility in a liquid formulation.

### Background to the Invention

During the industrial production of ethylenically unsaturated monomers there is usually a requirement for high temperature processing operations, such as purification by fractional distillation, and the performance of said operations at elevated temperatures can often cause unwanted thermal polymerisation of the monomer. The thus formed polymer is a problem economically, and can also give rise to safety and process efficiency issues which adversely affect the monomer processing operation.

Economic loss occurs because the yield of valuable monomer is reduced due to polymerisation of the monomer to form a polymer which is merely valued, at best, on its calorific content as a fuel, whilst the process efficiency and safety problems are associated with the tendencies of the polymer to foul surfaces such as heat transfer surfaces, and to cause blockages to flow through the equipment or to increase the viscosity of process streams, so that flux oil or valuable monomer has to be added in order to reduce the viscosity and thereby enable the process stream to be moved readily by gravity, or by forced flow such as by pumping.

In order to control such unwanted polymerisation it is common practice to employ an antipolymerant composition in the process stream. These anti-polymerant compositions essentially fall into one of two categories, namely inhibitors and retarders.

Inhibitors are effective in preventing the formation of polymer, but they are substantially consumed in this process. This consumption can cause problems in situations where the inhibitor cannot be replenished, for example because of feed pump failure. Consequent to the inhibitor being consumed there is a loss of the means of control of the polymerisation and, thus, rapid polymerisation can then occur.

Retarders, on the other hand, may be less effective than inhibitors in limiting the amount of polymerisation, but they do have the advantage that they are not substantially consumed under their conditions of use. Retarders are, therefore, more reliable because they are longer acting and they provide some security in situations where unplanned circumstances arise.

The production and processing of ethylenically unsaturated monomers is a continuous process and, as such, requires constant addition of the antipolymerant to the process in order to effectively prevent unwanted polymerisation. In order to achieve this effectively, the antipolymerant is constantly injected into the process stream at the desired location(s) as a liquid formulation. At normal handling temperatures, the vast majority of antipolymerants used are in the solid state and, therefore, cannot be directly injected into the process. Thus, in order to effectively dose the inhibitor into the process stream, the antipolymerant is either heated above its melting point to produce an injectable liquid or, more commonly, is dissolved in a suitable solvent. Suitable solvents are selected on the basis of the monomer being processed and include the monomer itself, liquids that are already present in the process stream (e.g. methanol in the production of methyl methacrylate), or solvents that are compatible with the monomer process stream. Compatible solvents are typically those that are chemically unreactive with components in the monomer process stream, do not interfere with physical processes during the monomer purification process, and are of significantly low volatility such that they do not co-distil with the final monomer product. Examples of suitable solvents may include water and various glycols.

In general, antipolymerants are supplied to the monomer manufacturer in suitable solvents at concentrations of typically 10 percent by weight and above. This is in order to reduce the volume of material required for transport to the monomer production facility, thereby reducing cost and transport frequency of generally hazardous materials. However, some antipolymerants are of sufficiently low solubility in a suitable solvent as to make the supply of a liquid formulation impractical. In such cases the antipolymerant may be supplied as the solid active to the monomer production plant. Typically, the antipolymerant is then dissolved in a suitable solvent, at low concentration, at the monomer production plant prior to dosing into the process stream. This is generally a batch process operation, involving the preparation of a stock antipolymerant solution every few days. As such, considerable manual handling and unnecessary potential exposure to hazardous chemicals may be involved.

Examples of such procedures include the use of either phenothiazine or hydroquinone as an inhibitor for (meth)acrylic acid and its esters, the use of hydroquinone in acrylonitrile and the use of benzoquinone in vinyl acetate monomer. In these examples, the operation may involve dissolving the solid inhibitor in a batch process in a suitable solvent, such as the monomer, to produce a low concentration (typically <5 percent by weight) inhibitor stock solution. The solid inhibitor may be added to the solvent in one portion using a mechanical system, such as a hopper, or via a manual handling operation. The latter process generally involves one or more process operators manually charging multiple containers of solid antipolymerant into the stock solution vessel.

Either of these processes involves considerable drawbacks when compared to supplying a concentrated liquid antipolymerant formulation. The use of a mechanical system to add the solid antipolymerant to the solvent involves additional capital costs and potentially results in the increased exposure of plant personnel to both the antipolymerant and solvent. Adding the antipolymerant manually also increases the risk of exposure to potentially hazardous chemicals, as well as incurring additional labour costs.

Despite the low solubility of the aforementioned antipolymerants, they are still used extensively due to their high activity in preventing unwanted polymerisation. It would, therefore, be advantageous to supply such poorly soluble antipolymerants in a liquid form at high concentration for direct addition to an ethylenically unsaturated monomer production process. This would result in a substantial reduction in the risk of exposure for operations personnel, and would reduce the costs associated with solid handling processes.

One method of providing a liquid formulation of a poorly soluble compound is through the preparation of a suspension concentrate (SC). Suspension concentrates (SCs) are prepared by forming a stable dispersion of one or more solid active components in a suitable liquid media (carrier), which may be either aqueous or non-aqueous. The solid active components are first processed to provide solid particles of a small (micrometre) uniform size. To the solid is then added at least one dispersing agent and at least one thickening agent (inorganic or organic) in order to control the viscosity of the SC in the liquid carrier. In addition, anti-foaming agents and preservatives may also be added to the formulation.

SC formulations have been widely used in certain sectors of the chemical industry, particularly the agrochemical industry, as a method of providing poorly soluble active compounds as a liquid formulation. Thus, WO-A-2006/003371 describes the preparation of a formulation for a range of selective herbicides that possess very low solubilities in water. Stable liquid formulations were prepared in water by providing a suspension concentrate of the active herbicide at concentrations of up to 600g/L which could then be delivered to the end user for further dilution. In addition, WO-A-2011/051969 teaches a method of providing a stable aqueous formulation of one non-selective and one selective herbicide. The prepared SC exhibited very high physical stability at high active concentrations. In a similar process to that described previously, SCs can also be prepared in non-aqueous media. Thus, US-A-2006/276337 discloses the preparation of an organic suspension concentrate to supply a high concentration of a sulfonylurea herbicide in a liquid form.

In addition to the agrochemical industry, SCs have also found use in other chemical sectors. For example, US-A-2011/118163 teaches the application of a suspension concentrate to provide a stable liquid formulation as a fragrance delivery system, whilst US-B-6410543 describes the preparation of a suspension concentrate to provide a liquid delivery system for antimicrobial agents for treatment of livestock. In a similar application, NZ-A-333497 relates to the use of a suspension concentrate for delivery of antibiotic agents in animal treatments.

Furthermore, the use of dispersant chemicals is well known in the petrochemical industry. Such materials are often applied as antifoulants in order to prevent polymeric deposits from adhering to the surfaces of production equipment, thereby reducing the possibility of equipment blockages and, as a consequence, increasing the time periods between maintenance shutdowns. Thus, US-A-2010/130385 describes the use of a formulation containing a polymeric surfactant as a paraffin inhibitor in oil production processes, wherein the additive prevents the formation of paraffin deposits and the potential solidification of the oil, whilst WO-A-2010/059770 is concerned with the use of a styrene sulphonate polymer as an antifoulant additive for acrylonitrile manufacture, the additive preventing foulant species from adhering to the surfaces of the process equipment. Also, WO-A-2009/108566 discloses the use of an antifoulant composition to reduce fouling in furnaces used in chemical manufacture; the claimed composition includes an alpha-olefin maleic anhydride copolymer to disperse coke and asphaltene precipitates from the furnace surfaces.

Such antifoulant compositions have previously been used in combination with polymerisation inhibitors. Thus, for example, CN-A-101838058 teaches the use of a polymerisation inhibitor and dispersant to prevent scale formation in process water system for an ethylene production process and CN-A-101358144 describes the use of a combination of an antioxidant, a polymerisation inhibitor and a dispersant for use in an alkaline wash process during a hydrocarbon treatment process, whilst JP-A-2005248055 discusses the preparation of an oxidation inhibitor dispersed in water.

However, none of these prior art compositions considers the use of SCs as a vehicle for the supply of antipolymerants in ethylenically unsaturated monomer production processes, or suggests a method of supplying a poorly soluble antipolymerant in a liquid form suitable for direct use in such processes, and it is this problem which the present invention seeks to address.

Efficacy and ease of use are the desired attributes of a successful composition for the control of the degree of polymerisation of ethylenically unsaturated monomers during their processing. In many circumstances, the ease of use is gained by providing the antipolymerant in a liquid composition at a sufficiently high concentration such that no further processing is required prior to its use in the process. However, some antipolymerants have such low solubility characteristics as to render their supply in a solvent system uneconomical; for example, high transport costs may be involved in providing dilute solutions of the active component(s). In such circumstances, the antipolymerant is supplied as the solid, and this is diluted in a suitable solvent at the location of treatment. However, as previously observed, such procedures involve unwelcome manual handling of the solid antipolymerant, thereby increasing the risk of operator exposure to chemical contact.

Thus, the desired characteristics of such a composition include low potential for harm to humans, as well as high efficacy of control of the amount of polymerisation under the process conditions. The conditions which prevail during the industrial processing of ethylenically unsaturated monomers can involve the use of elevated temperatures, for example up to about 140°C, for extended periods, e.g. two hours or more, and with low or very low oxygen levels.

Furthermore, due to the complex nature of such processing plants, non-standard operating conditions of temperature, dwell time and oxygen content can occur from time to time, thereby resulting in greater than normal reliance on the polymerisation control composition. The non-standard conditions can also involve an interruption in the flow of the polymerisation control composition into the ethylenically unsaturated monomer process stream - which may occur, for example, because a distillation column needs to be run under total reflux for a period of time. In such an event, it is a requirement of the polymerisation control composition that its efficacy should not be quickly exhausted, but that it should continue to provide control over the whole duration of the period of the non-standard operating conditions. Such long-lasting polymerisation control properties can be provided by a retarder composition, whereas inhibitors do not have such longevity of efficacy and can be found to become ineffective too quickly to be useful.

### Summary of the Invention

The present inventors have, therefore, sought to provide a formulation and method which allow for the efficient delivery of antipolymerants of low solubility into processes for the production of ethylenically unsaturated monomers, and associated process streams, and which avoid the potential health and safety problems and economic disadvantages which are associated with the methods of the prior art and facilitate the safe and efficient production of the said monomers.

Hence, the inventors have developed a method for supplying poorly soluble antipolymerants in a concentrated liquid form that can be directly supplied into a process for the production of ethylenically unsaturated monomers and have provided a concentrated liquid formulation in the form of a suspension concentrate (SC) that does not require further onsite manipulation. It is found that the antipolymerant components of the said liquid formulations are highly active in preventing unwanted polymerisation of ethylenically unsaturated monomers during processing operation(s), and the method of the present invention effectively allows for the removal of many of the manual operations that would otherwise be required if the active components were supplied in their native solid state.

Thus, according to a first aspect of the present invention there is provided an antipolymerant composition for the prevention of unwanted polymerisation reactions during the production and processing of ethylenically unsaturated compounds, said composition comprising a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier,
   wherein said phenol compounds are hydroquinone compounds of the general formula (i-a): 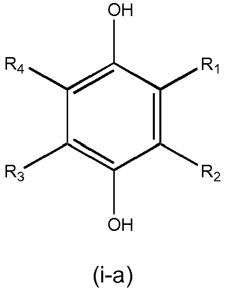 wherein:
   R₁ to R₄ are each independently selected from the group consisting of H, C₁, C₂, C₃, C₄, C₅, C₆, C₇ or C₈ straight or branched chain saturated or unsaturated hydrocarbons and optionally substituted phenyl and benzyl.

In certain embodiments of the invention, said at least one dispersing agent may comprise an ionic or non-ionic surfactant.

Optionally, said composition additionally comprises at least one thickening agent.

Optionally, said composition additionally comprises at least one preservative agent.

Optionally, said composition additionally comprises at least one stable free radical compound. Stable, in the context of the present invention, refers to the compound being chemically and physically robust, and not subject to decomposition, under normal storage conditions, i.e. under atmospheric conditions, at ambient temperature and pressure. Examples of suitable compounds include, but are not limited to, 2,2,6,6-tetramethyl piperidine-1-oxyl compounds (TEMPOs).

In said compositions, the weight content of component (a) may be in the range of from 10:90 to 90:10, but is typically in the range of from 30:70 to 70:30, relative to the remaining components in the composition.

Ethylenically unsaturated compounds in the context of the present invention may, for example, comprise vinyl aromatic monomers or other ethylenically unsaturated monomers.

According to a second aspect of the present invention, there is provided a process stream which comprises an antipolymerant composition in combination with at least one ethylenically unsaturated compound, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier.

Typically, said at least one ethylenically unsaturated compound comprises a vinyl aromatic monomer or other ethylenically unsaturated monomer.

In said process streams, embodiments of the invention envisage that the weight ratio of antipolymerant composition (comprising components (a), (b), (c) and (d)) to ethylenically unsaturated compound is in the range of from 1:200 to 1:40000.

According to a third aspect of the present invention, there is provided a method for the prevention of unwanted polymerisation reactions during the production and processing of ethylenically unsaturated compounds, said method comprising treating a composition comprising at least one ethylenically unsaturated compound with an antipolymerant composition, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier.

The invention also envisages the use of an antipolymerant composition for the prevention of unwanted polymerisation reactions during the production and processing of ethylenically unsaturated compounds, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier.

### Description of the Invention

As hereinbefore defined, the present invention provides compositions comprising at least one antipolymerant selected from the group consisting of phenols, quinones, hydroxylamines, thiazines and aromatic amines, wherein said phenols are hydroquinone compounds of the general formula (i-a): 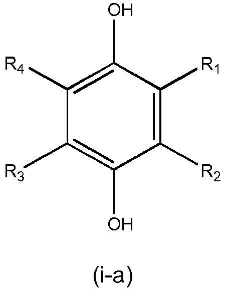 wherein:
R₁ to R₄ are each independently selected from the group consisting of H, C₁, C₂, C₃, C₄, C₅, C₆, C₇ or C₈ straight or branched chain saturated or unsaturated hydrocarbons and optionally substituted phenyl and benzyl.

Preferred hydroquinones for use in the context of the present invention are 2,5-di-alkyl substituted hydroquinones and, most preferably, the di-substituted hydroquinone is 2,5-di-tert-butylhydroquinone.

The antipolymerant is provided in a liquid composition and is dispersed therein as a solid in a suitable carrier liquid by formulating with at least one dispersing agent and, optionally, at least one thickening agent. The compositions optionally also comprise at least one stable free radical compound.

The invention also envisages process streams which additionally comprise at least one ethylenically unsaturated compound, typically comprising at least one vinyl aromatic monomer or other ethylenically unsaturated monomer.

The invention further provides a method by which antipolymerant(s) of low solubility may be supplied to ethylenically unsaturated monomers without the requirement to manually handle the solid antipolymerant(s). Thus, the solid antipolymerant is provided as a suspension concentrate in a suitable solvent for injection into the process, thereby facilitating the reduction of unwanted polymerisation of the ethylenically unsaturated monomer.

Suitable quinones for use in the context of the present invention are of the general formula (i-b): 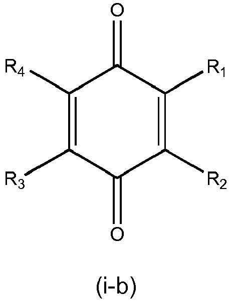 wherein:
R₁ to R₄ are each independently selected from the group consisting of H, C₁, C₂, C₃, C₄, C₅, C₆, C₇ or C₈ straight or branched chain saturated or unsaturated hydrocarbons and optionally substituted phenyl and benzyl.

Preferred quinones for use in the context of the present invention are 2,5-di-alkyl substituted quinones and, most preferably, the di-substituted quinone is 2,5-di-tert-butyl-p-benzoquinone.

In certain embodiments of the invention, hydroxylamines for use in the compositions and method of the present invention are typically selected from the group of compounds (ii): 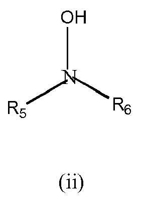 wherein:
R₅ to R₆ are each independently selected from the group consisting of C₁, C₂, C₃, C₄, C₅, C₆, C₇ or C₈ straight or branched chain saturated or unsaturated hydrocarbons or hydroxyhydrocarbons and C₅, C₆ or C₇ saturated or unsaturated hydrocarbon rings which may optionally be substituted rings, and arylakyls which may optionally be substituted on the aryl moiety, and wherein the alkyl moiety comprises C₁, C₂, C₃ or C₄ straight or branched chain hydrocarbons, and wherein the aryl moiety comprises one or more rings which are optionally substituted and, in the case of more than one ring, these may include fused rings.

Hydroxylamines which are particularly useful in the compositions according to the first aspect of the invention include aliphatic hydroxylamines such as bis(hydroxypropyl) hydroxylamine. Preferred hydroxylamines, however, are aromatic hydroxylamines, and a particularly preferred aromatic hydroxylamine is N,N-dibenzyl hydroxylamine.

Dispersing agents which are especially useful in the context of the present invention are typically selected from at least one ionic or non-ionic surfactant and include, but are not limited to, polyacrylate esters and polyarylphenylether salts.

Carrier liquids for use in the compositions, process streams and methods of the present invention are selected from both polar and non-polar liquids and typically include, for example, water, alcohols, glycols, ketones, aldehydes, aromatic hydrocarbons and alkanes.

Compositions according to the first aspect of the present invention may also optionally contain one or more thickening agents. Suitable thickening agents for use in this context include, but are not limited to, xanthan gum and guar gum.

Compositions according to the invention may also optionally comprise at least one preservative agent. Typical examples of suitable preservative agents include, but are not limited to, 2-methyl-4-isothiazolin-3-one, 5-chloro-2-methyl-4-isothiazolin-3-one, 1,2-benzisophiazolin-3-one, 2,2'-methylenebis(5-chlorophenol), 2-bromo-2-nitropropane-1,3-diol and 1,2-octandiol.

Compositions of the present invention may also optionally contain one or more free radical compounds which most conveniently comprise nitroxyl compounds of formula (iii): 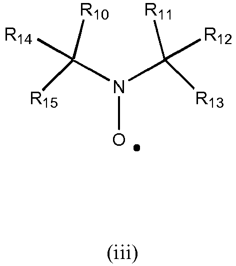 wherein:
R₁₀ to R₁₅ are each independently selected from the group consisting of C₁, C₂, C₃, C₄, C₅, C₆, C₇ or C₈ straight or branched chain saturated or unsaturated hydrocarbons and C₅, C₆ or C₇ saturated or unsaturated hydrocarbon rings, and R₁₀ plus R₁₁ may together form a saturated or unsaturated ring optionally containing a further hetero atom, wherein the ring is optionally further substituted with one or more branched or straight chain alkyl or alkenyl groups or one or more moieties selected from hydroxyl, oxyl, amino and alkoxyl, and wherein two or more such nitroxyl containing rings may be joined by any linking groups.

Examples of suitable linking groups include C₁ to C₁₈ alkylene, C₄ to C₁₈ alkenylene, xylylene, a divalent acyl radical of an aliphatic, araliphatic or aromatic dicarboxylic acid or of a dicarbamic acid or of a phosphorous-containing acid or of a sulphur containing acid or a bivalent silyl radical or a sulphone, a sulphide or a nitrogen containing group or a bisether.

Preferred linking groups include, for example, an acyl radical of an aliphatic C₂ to C₃₆ dicarboxylic acid, or of a C₈ to C₁₄ cycloaliphatic acid or of a C₈ to C₁₄ aromatic dicarboxylic acid or of a C₈ to C₁₄ aromatic dicarbamic acid.

Preferred nitroxyl compounds are 4-hydroxy-2,2,6,6-tetramethylpiperidin-N-oxyl and 4-oxo-2,2,6,6-tetramethylpiperidin-N-oxyl.

It is generally desirable that the particle sizes of the suspended materials in the suspension concentrate formulations of the present invention should be less than 10 microns.

The compositions of the present invention find application in the prevention of unwanted polymerisation reactions during the production and processing of ethylenically unsaturated compounds, typically comprising vinyl aromatic monomers or other ethylenically unsaturated monomers, and are suitably added to a process stream thereof. Typical vinyl aromatic monomers which are present in such process streams include, for example, styrene monomer, divinyl benzene and the like. Other ethylenically unsaturated monomers include acrylonitrile, (meth)acrylic acid and esters thereof, butadiene, isoprene and the like.

The method of the present invention may be carried out by any of the standard techniques well known in the art, for example by injecting into a process stream one or more antipolymerants, either separately or together, e.g. by premixing one antipolymerant as a separate suspension concentrate with another antipolymerant, either provided in a suspension concentrate or added by conventional means (i.e. in suitable solvent).

The composition and method of the present invention will now be further illustrated, though without in any way limiting the scope of the invention, by reference to the following examples.

### Examples

### General outline of procedure for preparation of a suspension concentrate formulation

To the liquid carrier (approximately 80% of the required amount) is added the required quantity of the dispersing agent and the mixture is stirred until a fully homogeneous solution is formed. Under high shear conditions, the desired amount of active inhibitor component is added to the dispersant solution and the formulation is stirred until it becomes completely homogeneous. The resulting dispersed active inhibitor is transferred to a bead mill where it is continuously milled until the required particle size distribution is obtained. Desirably, the particle size is less than 10 microns. Under additional stirring, the remaining liquid carrier, optionally a preservative agent and optionally a thickening agent are added to the milled formulation until a homogeneous suspension concentrate is formed. The required quantities of the components of the formulation are in each case determined with reference to the scale of the process which is being conducted.

### Concentration of actives in liquid carrier

In order to highlight the increased antipolymerant content that can be achieved, the solubility of three antipolymerants in ethylbenzene, a widely used solvent for supplying antipolymerants for styrene monomer production, is compared with concentration when the antipolymerant is supplied as a SC. The comparative data for the antipolymerants are set out in Table 1.

**TABLE 1 MAXIMUM CONCENTRATION OF ACTIVES IN LIQUID CARRIER**

| Antipolymerant | Solubility in ethylbenzene (w/w%) | Concentration in SC (w/w%) |
|---|---|---|
| 2,5-di-tert-butyl-p-benzoquinone | 11.9 | 45.0 |
| 2,5-di-tert-butyl hydroquinone | 6.7 | 45.0 |
| N, N-dibenzyl hydroxylamine | 1.6 | 45.0 |

Thus, it is clear that, for all three active components, the concentration achieved in the SC is far greater than that observed in ethylbenzene. The same concentration could also be obtained when two of the active components were combined in the SC. By contrast, combining more than one of the actives in ethylbenzene, the total concentration of actives was reduced. Thus, for a combination of 2,5-di-tert-butyl-p-benzoquinone and dibenzylhydroxylamine, the total concentration in ethylbenzene was <10 w/w%.

This effect is further illustrated by reference to a further example wherein a stable suspension concentrate of phenothiazine (PTZ), containing 40 percent by weight of the PTZ component, was easily obtained using the above methodology. PTZ is a widely used inhibitor in (meth)acrylic acid and ester processes, but has low solubility in most solvents that are compatible with such processes. In most industrial cases, PTZ is supplied in its solid form and dissolved in the monomer to form a dilute solution for dosing into the monomer purification process. Thus, for example, in laboratory tests, PTZ had the following solubilities: acrylic acid (2.6 w/w%), methacrylic acid (2.4 w/w%), n-butyl acrylate (9.7 w/w%), methyl methacrylate (8.4 w/w%). Hence, all of the above solutions have significantly lower loadings of active PTZ compared to the suspension concentrate.

### Laboratory efficacy tests

Laboratory tests were carried out in order to demonstrate the effectiveness of various individual substances and compositions thereof for controlling the amount of unwanted polymerisation of an ethylenically unsaturated compound. The particular ethylenically unsaturated compound used in the tests was a vinyl aromatic monomer, specifically styrene monomer. The use of a vinyl aromatic monomer for this purpose is illustrative and is not intended to exclude other ethylenically unsaturated compounds from the scope of this invention.

Batch tests (the results of which are detailed in Table 2), and continuous flow tests were carried out in order to represent different types of processing conditions which can occur in the industrial processing of ethylenically unsaturated compounds. These tests compare the compositions of the present invention with compositions of the prior art. DNBP (2,4-dinitro-ortho-sec-butyl-phenol) is the polymerisation retarder of the prior art most commonly employed in commercial production of vinyl aromatic monomers.

The following abbreviations are used hereinafter in Tables 2 and 4:
DNBP = 4,6-Dinitro-2-sec-butyl phenol
HQ = 2,5-di-tert-butyl hydroquinone
BQ = 2,5-di-tert-butyl-p-benzoquinone
DBHA = N,N-dibenzyl hydroxylamine

### Batch Tests

The batch test was designed in order to show whether a test composition is an inhibitor or a retarder. The tests were carried out in styrene monomer refluxing under reduced pressure at 120°C in order to reduce the presence of atmospheric oxygen to such low levels as can be expected in a commercial styrene monomer purification plant.

For the batch tests the vinyl aromatic monomer composition containing the test substances was heated to 120°C and stirred under reduced pressure so that it refluxed at the test temperature. Samples were taken at intervals of time and tested for polymer content in order to assess the effectiveness of polymerisation control. The results are presented in Table 2.

**TABLE 2 BATCH TESTS**

| **Test No.** | **Component 1 (ppm)** | **Component 2 (ppm)** | **Polymer (ppm) @ (time)** | | | **Effect** |
|---|---|---|---|---|---|---|
| | | | 30 mins | 150 mins | 180 mins | |
| **Control 1** | DNBP (600) | | | | 4000 | Retarder |

| | **Component 1** | **Component 2** | | | | |
|---|---|---|---|---|---|---|
| **Control 2** | BQ (200) | DBHA (400) | | | 3500 | Retarder |
| **Control 3** | HQ (400) | DBHA (200) | | | 9500 | Retarder |
| **1** | BQ (200) | DBHA (400) | | | 7000 | Retarder |
| **2** | HQ (400) | DBHA (200) | | | 296 | Retarder |

The results set out in Table 2 confirm that DNBP (Control 1) has long term effectiveness and is therefore a retarder.

Controls 2 and 3 involve the active components of two retarder compositions. The components were added to the test in the form of dilute solutions (2 w/w%) in styrene and diethylene glycol monobutyl ether (DEGMBE). The results show that both the benzoquinone/hydroxylamine and hydroquinone/hydroxylamine combinations are effective as polymerisation retarders for styrene. However, the active components in these compositions have limited solubility in solvents that are compatible with the process stream, such as styrene, ethylbenzene or DEGMBE. Thus, the components could only be supplied as dilute solutions in these solvents, leading to increased costs associated with transporting and storing large volumes of solvent.

Tests 1 and 2 involve the same compositions tested in controls 2 and 3. However, in these tests the active components were added to the system as a liquid suspension concentrate formulation. There is little effect on the activity of the components when they are added to the system as a suspension concentrate, demonstrating the effectiveness of the present invention in delivering effective, poorly soluble antipolymerants to a monomer system. Indeed, both the SC formulations have similar activity to that of DNBP, the material most commonly employed in the vinyl aromatic monomer processing industry.

### Acrylic acid efficacy test

In order to further demonstrate the use of suspension concentrates as a method for providing an efficacious liquid formulation for a poorly soluble inhibitor, a process was carried out wherein a suspension concentrate formulation of PTZ was used as an inhibitor for acrylic acid polymerisation. In a typical test, a series of tubes containing acrylic acid and inhibitor was sparged with a lean air mixture (5% oxygen in nitrogen). These tubes were then heated at 120°C until the first signs of polymerisation were detected (by means of the formation of white precipitate), and this was determined as the inhibition period. Table 3 shows a comparison of the inhibition period of a PTZ suspension concentrate with the solid material, on an equivalent active dosage.

**TABLE 3 ACRYLIC ACID TUBE TEST**

| Inhibitor (ppm) | Inhibition Period (min) |
|---|---|
| None | <10 |
| PTZ solid (25) | 531 |
| PTZ SC (25) | 543 |

Table 3 shows that the PTZ suspension concentrate offers an equivalent performance to that of the solid inhibitor but obviates the requirement for handling of the solid material.

Furthermore, the compositions of the present invention do not contain substances which are toxic to humans and, consequently, they are of considerable benefit when compared to the formulations of the prior art that typically contain dinitrophenols such as DNBP which, as previously noted, is the material most commonly used in the vinyl aromatic monomer processing industry. A comparison of the properties of the substances utilised in the compositions of the current invention with those of the prior art material DNBP is provided in Table 4.

**TABLE 4 COMPARISON OF HAZARD CLASSIFICATIONS**

| | **DNBP** | **BQ** | **HQ** | **DBHA** |
|---|---|---|---|---|
| Oral Toxicity | Yes | No | No | No |
| Dermal Toxicity | Yes | No | No | No |
| Toxic by Inhalation | Yes | No | No | No |

Thus, according to these hazard classifications, it is apparent that the components of the compositions of the present invention pose much less hazard to humans than does DNBP. As a consequence, the need to control the exposure of plant operators can be greatly reduced by using the compositions of the present invention instead of DNBP during production and processing operations using vinyl aromatic monomers. Hence, the compositions of the present invention provide both a more effective and a safer means of controlling unwanted polymerisation of vinyl aromatic monomers when compared to the compositions of the prior art.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

## Claims

1. An antipolymerant composition for the prevention of unwanted polymerisation reactions during the production and processing of ethylenically unsaturated compounds, said composition comprising a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier,
wherein said phenol compounds are hydroquinone compounds of the general formula (i-a): 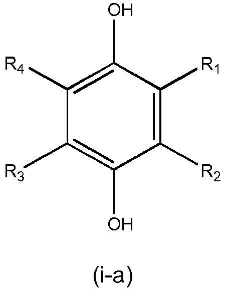 wherein:
R₁ to R₄ are each independently selected from the group consisting of H, C₁, C₂, C₃, C₄, C₅, C₆, C₇ or C₈ straight or branched chain saturated or unsaturated hydrocarbons and optionally substituted phenyl and benzyl,
wherein said hydroquinones are optionally selected from 2,5-di-alkyl substituted hydroquinones, optionally 2,5-di-tert-butylhydroquinone.

2. A composition as claimed in claim 1 wherein said quinones are of the general formula (i-b): 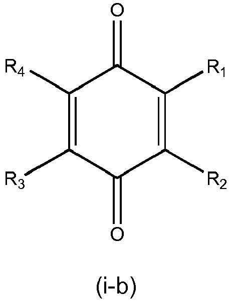 wherein:
R₁ to R₄ are each independently selected from the group consisting of H, C₁, C₂, C₃, C₄, C₅, C₆, C₇ or C₈ straight or branched chain saturated or unsaturated hydrocarbons and optionally substituted phenyl and benzyl,
wherein said quinones are optionally selected from 2,5-di-alkyl substituted quinones, optionally 2,5-di-tert-butyl-p-benzoquinone.

3. A composition as claimed in claim 1 or 2 wherein said hydroxylamines are selected from the group of compounds (ii): 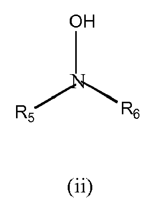 wherein:
R₅ to R₆ are each independently selected from the group consisting of C₁, C₂, C₃, C₄, C₅, C₆, C₇ or C₈ straight or branched chain saturated or unsaturated hydrocarbons or hydroxyhydrocarbons and C₅, C₆ or C₇ saturated or unsaturated hydrocarbon rings which may optionally be substituted rings, and arylakyls which may optionally be substituted on the aryl moiety, and wherein the alkyl moiety comprises C₁, C₂, C₃ or C₄ straight or branched chain hydrocarbons, and wherein the aryl moiety comprises one or more rings which are optionally substituted and, in the case of more than one ring, these may include fused rings,
wherein said hydroxylamines are optionally selected from aliphatic hydroxylamines and aromatic hydroxylamines, optionally bis(hydroxypropyl) hydroxylamine or N,N-dibenzyl hydroxylamine.

4. A composition as claimed in claim 1, 2 or 3 which additionally comprises at least one thickening agent, wherein said thickening agents are optionally selected from xanthan gum and guar gum.

5. A composition as claimed in any one of claims 1 to 4 wherein said composition additionally comprises at least one preservative agent, wherein said preservative agents are optionally selected from 2-methyl-4-isothiazolin-3-one, 5-chloro-2-methyl-4-isothiazolin-3-one, 1,2-benzisophiazolin-3-one, 2,2'-methylenebis(5-chlorophenol), 2-bromo-2-nitropropane-1,3-diol and 1,2-octandiol.

6. A composition as claimed in any preceding claim which additionally comprises at least one stable free radical compound, wherein said at least one free radical compound optionally comprises at least one nitroxyl compound of formula (iii): 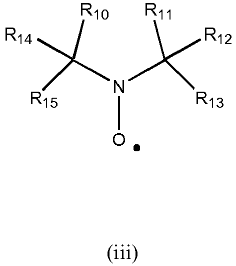 wherein:
R₁₀ to R₁₅ are each independently selected from the group consisting of C₁, C₂, C₃, C₄, C₅, C₆, C₇ or C₈ straight or branched chain saturated or unsaturated hydrocarbons and C₅, C₆ or C₇ saturated or unsaturated hydrocarbon rings, and R₁₀ plus R₁₁ may together form a saturated or unsaturated ring optionally containing a further hetero atom, wherein the ring is optionally further substituted with one or more branched or straight chain alkyl or alkenyl groups or one or more moieties selected from hydroxyl, oxyl, amino and alkoxyl, and wherein two or more such nitroxyl containing rings may be joined by any linking groups.

7. A composition as claimed in claim 6 wherein said linking groups include C₁ to C₁₈ alkylene, C₄ to C₁₈ alkenylene, xylylene, a divalent acyl radical of an aliphatic, araliphatic or aromatic dicarboxylic acid or of a dicarbamic acid or of a phosphorous-containing acid or of a sulphur containing acid or a bivalent silyl radical or a sulphone, a sulphide or a nitrogen containing group or a bisether, wherein said linking groups are optionally selected from an acyl radical of an aliphatic C₂ to C₃₆ dicarboxylic acid, or of a C₈ to C₁₄ cycloaliphatic acid or of a C₈ to C₁₄ aromatic dicarboxylic acid or of a C₈ to C₁₄ aromatic dicarbamic acid.

8. A composition as claimed in claim 6 or 7 wherein said nitroxyl compound is selected from 2,2,6,6-tetramethyl piperidine-1-oxyl compounds (TEMPOs), and is optionally selected from 4-hydroxy-2,2,6,6-tetramethylpiperidin-N-oxyl and 4-oxo-2,2,6,6-tetramethylpiperidin-N-oxyl.

9. A composition as claimed in any preceding claim wherein said dispersing agents are selected from at least one ionic or non-ionic surfactant, and optionally selected from polyacrylate esters and polyarylphenylether salts.

10. A composition as claimed in any preceding claim wherein said carrier liquids are selected from polar and non-polar liquids, and optionally selected from water, alcohols, glycols, ketones, aldehydes, aromatic hydrocarbons and alkanes.

11. A composition as claimed in any preceding claim wherein the weight content of component (a) is in the range of from 30:70 to 70:30 relative to the remaining components in the composition.

12. A composition as claimed in any preceding claim wherein the particle sizes are less than 10 microns.

13. A process stream comprising an antipolymerant composition in combination with at least one ethylenically unsaturated compound, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier,
wherein said at least one ethylenically unsaturated compound optionally comprises a vinyl aromatic monomer or other ethylenically unsaturated monomer, wherein said vinyl aromatic monomer is optionally styrene or divinyl benzene and said ethylenically unsaturated monomer is optionally selected from acrylonitrile, (meth)acrylic acid and esters thereof, butadiene and isoprene, and/or wherein the weight ratio of antipolymerant composition to ethylenically unsaturated compound is optionally in the range of from 1:200 to 1:40000.

14. A method for the prevention of unwanted polymerisation reactions during the production and processing of ethylenically unsaturated compounds, said method comprising treating a composition comprising at least one ethylenically unsaturated compound with an antipolymerant composition, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier.
wherein, optionally, at least one antipolymerant is prepared as a suspension concentrate and said suspension concentrate is injected into a process stream.

15. The use of an antipolymerant composition for the prevention of unwanted polymerisation reactions during the production and processing of ethylenically unsaturated compounds, wherein said antipolymerant composition comprises a concentrated liquid formulation, wherein said concentrated liquid formulation is a suspension concentrate which comprises:
(a) at least one compound selected from the group comprising phenols, quinones, thiazines, hydroxylamines and aromatic amines;
(b) at least one dispersing agent; and
(c) at least one polar or non-polar liquid carrier.

## Patentansprüche

1. Antipolymerisationszusammensetzung zur Verhinderung von unerwünschten Polymerisationsreaktionen während der Herstellung und Verarbeitung von ethylenisch ungesättigten Verbindungen, wobei die Zusammensetzung eine konzentrierte flüssige Formulierung umfasst, wobei die konzentrierte flüssige Formulierung ein Suspensionskonzentrat ist, das Folgendes umfasst:
(a) mindestens eine Verbindung ausgewählt aus der Gruppe umfassend Phenole, Chinone, Thiazine, Hydroxylamine und aromatische Amine;
(b) mindestens ein Dispergiermittel; und
(c) mindestens einen polaren oder nicht polaren flüssigen Träger,
wobei die Phenolverbindungen Hydrochinonverbindungen der allgemeinen Formel (i-a) sind: 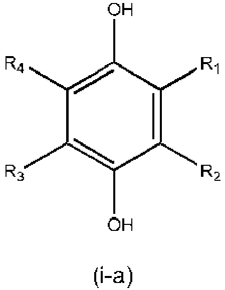 wobei:
R₁ bis R₄ jeweils unabhängig aus der Gruppe umfassend gerad- oder verzweigtkettige gesättigte oder ungesättigte H-, C₁-, C₂-, C₃-, C₄-, C₅-, C₆-, C₇- oder C₈-Kohlenwasserstoffe ausgewählt und optional substituiertes Phenyl und Benzyl sind,
wobei die Hydrochinone optional aus 2,5-Di-alkyl-substituierten Hydrochinonen, optional 2,5-Di-tert-Butylhydrochinon, ausgewählt sind.

2. Zusammensetzung gemäß Anspruch 1, wobei die Chinone von der allgemeinen Formel (i-b) sind: 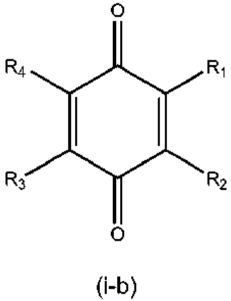 wobei:
R₁ bis R₄ jeweils unabhängig aus der Gruppe umfassend gerad- oder verzweigtkettige gesättigte oder ungesättigte H-, C₁-, C₂-, C₃-, C₄-, C₅-, C₆-, C₇- oder C₈-Kohlenwasserstoffe ausgewählt und optional substituiertes Phenyl und Benzyl sind,
wobei die Chinone optional aus 2,5-Di-alkyl-substituierten Chinonen, optional 2,5-Di-tert-butyl-p-benzochinon ausgewählt sind.

3. Zusammensetzung gemäß Anspruch 1 oder 2, wobei die Hydroxylamine aus der Gruppe der Verbindungen (ii) ausgewählt sind: 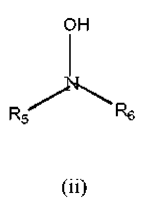 wobei:
R₅ bis R₆ jeweils unabhängig aus der Gruppe umfassend gerad- oder verzweigtkettige gesättigte oder ungesättigte C₁-, C₂-, C₃-, C₄-, C₅-, C₆-, C₇- oder C₈-Kohlenwasserstoffe oder Hydroxykohlenwasserstoffe und gesättigte oder ungesättigte C₅-, C₆- oder C₇-Kohlenwasserstoffringe, die optional substituierte Ringe aufweisen können, und Arylakyle, die optional an der Arylkomponente substituiert sein können, und wobei die Alkylkomponente gerad-oder verzweigtkettige C₁-, C₂-, C₃- oder C₄-Kohlenwasserstoffe umfasst, und wobei die Arylkomponente einen oder mehrere Ringe umfasst, die optional substituiert sind und die, im Fall von mehr als einem Ring, fusionierte Ringe einschließen können, ausgewählt sind,
wobei die Hydroxylamine optional aus aliphatischen Hydroxylaminen und aromatischen Hydroxylaminen, optional Bis(hydroxypropyl)hydroxylamin oder N,N-Dibenzylhydroxylamin, ausgewählt sind.

4. Zusammensetzung gemäß Anspruch 1, 2 oder 3, die zusätzlich mindestens ein Verdickungsmittel umfasst, wobei die Verdickungsmittel optional aus Xanthangummi und Guargummi ausgewählt sind.

5. Zusammensetzung gemäß einem der Ansprüche 1 bis 4, wobei die Zusammensetzung zusätzlich mindestens ein Konservierungsmittel umfasst, wobei die Konservierungsmittel optional aus 2-Methyl-4-isothiazolin-3-on, 5-Chlor-2-methyl-4-isothiazolin-3-on, 1,2-Benzisophiazolin-3-on, 2,2'-Methylenebis(5-chlorphenol), 2-Brom-2-nitropropane-1,3-diol und 1,2-Octandiol ausgewählt sind.

6. Zusammensetzung gemäß einem vorhergehenden Anspruch, die zusätzlich mindestens eine stabile Freie-Radikale-Verbindung umfasst, wobei die mindestens eine Freie-Radikale-Verbindung optional mindestens eine Nitroxylverbindung der Formel (iii) umfasst: 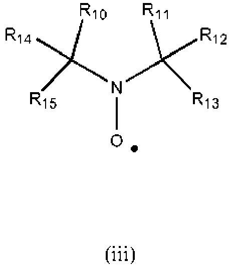 wobei:
R₁₀ bis R₁₅ jeweils unabhängig aus der Gruppe umfassend gerad- oder verzweigtkettige gesättigte oder ungesättigte C₁-, C₂-, C₃-, C₄-, C₅-, C₆-, C₇- oder C₈-Kohlenwasserstoffe und gesättigte oder ungesättigte C₅-, C₆- oder C₇-Kohlenwasserstoffringe ausgewählt sind, und R₁₀ plus R₁₁ zusammen einen gesättigten oder ungesättigten Ring bilden können, der optional ein weiteres Heteroatom enthalten kann, wobei der Ring optional weiter mit einer oder mehreren verzweigt- oder geradkettigen Alkyl- oder Alkenylgruppen oder einer oder mehreren Komponenten ausgewählt aus Hydroxyl, Oxyl, Amino und Alkoxyl substituiert ist, und wobei zwei oder mehrere solcher Nitroxyl enthaltenden Ringe durch beliebige Verkettungsgruppen zusammengefügt sein können.

7. Zusammensetzung gemäß Anspruch 6, wobei die Verkettungsgruppen C₁- bis C₁₈-Alkylen, C₄- bis C₁₈-Alkenylen, Xylylen, ein divalentes Acylradikal einer aliphatischen, araliphatischen oder aromatischen Dicarbonsäure oder einer Dicarbaminsäure oder einer Phosphor enthaltenden Säure oder einer Schwefel enthaltenden Säure oder ein bivalentes Silylradikal oder eine Sulfon, eine Sulfid oder eine Stickstoff enthaltende Gruppe oder ein Bisether einschließen, wobei die Verkettungsgruppen optional aus einem Acylradikal einer aliphatischen C₂- bis C₃₆-Dicarbonsäure oder einer cycloaliphatischen C₈- bis C₁₄-Säure oder einer aromatischen C₈- bis C₁₄-Dicarbonsäure oder einer aromatischen C₈- bis C₁₄-Dicarbaminsäure ausgewählt sind.

8. Zusammensetzung gemäß Anspruch 6 oder 7, wobei die Nitroxylverbindung aus 2,2,6,6-Tetramethylpiperidin-1-oxyl-Verbindungen (TEMPOs) ausgewählt ist und optional aus 4-Hydroxy-2,2,6,6-tetramethylpiperidin-N-oxyl und 4-Oxo-2,2,6,6-tetramethylpiperidin-N-oxyl ausgewählt ist.

9. Zusammensetzung gemäß einem vorhergehenden Anspruch, wobei die Dispergiermittel aus mindestens einem ionischen oder nicht ionischen Tensid ausgewählt sind und optional aus Polyacrylatestern und Polyarylphenylethersalzen ausgewählt sind.

10. Zusammensetzung gemäß einem vorhergehenden Anspruch, wobei die Trägerflüssigkeiten aus polaren und nicht polaren Flüssigkeiten ausgewählt sind und optional aus Wasser, Alkoholen, Glycolen, Ketonen, Aldehyden, aromatischen Kohlenwasserstoffen und Alkanen ausgewählt sind.

11. Zusammensetzung gemäß einem vorhergehenden Anspruch, wobei der Gewichtsanteil von Komponente (a) im Bereich von 30:70 bis 70:30 relativ zu den restlichen Komponenten in der Zusammensetzung liegt.

12. Zusammensetzung gemäß einem vorhergehenden Anspruch, wobei die Partikelgröße geringer als 10 Mikrometer ist.

13. Prozessstrom, umfassend eine Antipolymerisationszusammensetzung in Kombination mit mindestens einer ethylenisch ungesättigten Verbindung, wobei die Antipolymerisationszusammensetzung eine konzentrierte flüssige Formulierung umfasst, wobei die eine konzentrierte flüssige Formulierung ein Suspensionskonzentrat ist, das Folgendes umfasst:
(a) mindestens eine Verbindung ausgewählt aus der Gruppe umfassend Phenole, Chinone, Thiazine, Hydroxylamine und aromatische Amine;
(b) mindestens ein Dispergiermittel; und
(c) mindestens einen polaren oder nicht polaren flüssigen Träger,
wobei die mindestens eine ethylenisch ungesättigte Verbindung optional ein aromatisches Vinylmonomer oder anderes ethylenisch ungesättigtes Monomer umfasst, wobei das aromatische Vinylmonomer optional Styrol oder Divinylbenzol ist und das ethylenisch ungesättigte Monomer optional aus Acrylnitril, (Meth)acrylsäure und Estern davon, Butadien und Isopren ausgewählt ist, und/oder wobei das Gewichtsverhältnis der Antipolymerisationszusammensetzung zur ethylenisch ungesättigten Verbindung im Bereich von 1:200 bis 1:40000 liegt.

14. Verfahren zur Verhinderung von unerwünschten Polymerisationsreaktionen während der Herstellung und Verarbeitung von ethylenisch ungesättigten Verbindungen, wobei das Verfahren das Behandeln einer Zusammensetzung, die mindestens eine ethylenisch ungesättigte Verbindung umfasst, mit einer Antipolymerisationszusammensetzung umfasst, wobei die Antipolymerisationszusammensetzung eine konzentrierte flüssige Formulierung umfasst, wobei die konzentrierte flüssige Formulierung ein Suspensionskonzentrat ist, das Folgendes umfasst:
(a) mindestens eine Verbindung ausgewählt aus der Gruppe umfassend Phenole, Chinone, Thiazine, Hydroxylamine und aromatische Amine;
(b) mindestens ein Dispergiermittel; und
(c) mindestens einen polaren oder nicht polaren flüssigen Träger,
wobei, optional, mindestens ein Antipolymerisationsmittel als Suspensionskonzentrat hergestellt wird und das Suspensionskonzentrat in einen Prozessstrom injiziert wird.

15. Verwendung einer Antipolymerisationszusammensetzung zur Verhinderung von unerwünschten Polymerisationsreaktionen während der Herstellung und Verarbeitung von ethylenisch ungesättigten Verbindungen, wobei die Antipolymerisationszusammensetzung eine konzentrierte flüssige Formulierung umfasst, wobei die konzentrierte flüssige Formulierung ein Suspensionskonzentrat ist, das Folgendes umfasst:
(a) mindestens eine Verbindung ausgewählt aus der Gruppe umfassend Phenole, Chinone, Thiazine, Hydroxylamine und aromatische Amine;
(b) mindestens ein Dispergiermittel; und
(c) mindestens einen polaren oder nicht polaren flüssigen Träger.

## Revendications

1. Composition antipolymérante pour la prévention des réactions de polymérisation non désirées pendant la production et la transformation des composés éthyléniquement insaturés, ladite composition comprenant une formulation liquide concentrée, où ladite formulation liquide concentrée est un concentré de suspension qui comprend :
(a) au moins un composé choisi parmi le groupe comprenant les phénols, quinones, thiazines, hydroxylamines et amines aromatiques ;
(b) au moins un agent dispersant ; et
(c) au moins un liquide porteur polaire ou non polaire,
où lesdits composés phénoliques sont des composés d'hydroquinone de la formule générale (i-a) : 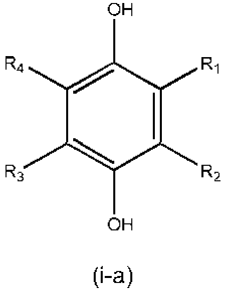 où:
R₁ à R₄ sont chacun choisis de manière indépendante parmi le groupe comprenant H, les hydrocarbures saturés ou insaturés, à chaîne droite ou ramifiée en C₁, C₂, C₃, C₄, C₅, C₆, C₇ ou C₈ et éventuellement le phényle substitué et le benzyle substitué,
où lesdites hydroquinones sont éventuellement choisies parmi les hydroquinones substituées 2,5-di-alkyliques, éventuellement la 2,5-di-tert-butylhydroquinone.

2. Composition selon la revendication 1, où lesdites quinones sont de la formule générale (i-b) : 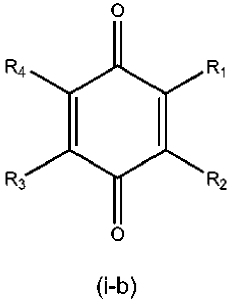 où:
R₁ à R4 sont chacun choisis de manière indépendante parmi le groupe comprenant H, les hydrocarbures saturés ou insaturés, à chaîne droite ou ramifiée en C₁, C₂, C₃, C₄, C₅, C₆, C₇ ou C₈ et éventuellement le phényle substitué et le benzyle substitué,
où lesdites quinones sont éventuellement choisies parmi les hydroquinones substituées 2,5-di-alkyliques, éventuellement la 2,5-di-tert-butyle-p-benzoquinone.

3. Composition selon la revendication 1 ou la revendication 2, où lesdites hydroxylamines sont choisies parmi le groupe de composés (ii) : 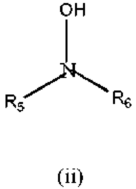 où:
R5 à R₆ sont chacun choisis de manière indépendante parmi le groupe comprenant les hydrocarbures ou hydroxyhydrocarbures saturés ou insaturés, à chaîne droite ou ramifiée en C₁, C₂, C₃, C₄, C₅, C₆, C₇ ou C₈ ou les anneaux hydrocarbures saturés ou insaturés en C₅, C₆ ou C₇, qui peuvent éventuellement être des anneaux substitués et les arylalkyles qui peuvent éventuellement être substitués sur la fraction aryle, et où la fraction alkyle comprend des hydrocarbures à chaîne droite ou ramifiée en C₁, C₂, C₃ ou C₄ et où la fraction aryle comprend un ou plusieurs anneaux qui sont éventuellement substitués et, dans le cas de plus d'un anneau, il peut s'agir d'anneaux fusionnés,
où lesdites hydroxylamines sont éventuellement choisies parmi les hydroxylamines aliphatiques et les hydroxylamines aromatiques éventuellement l'hydroxylamine bis (hydroxypropylique) ou l'hydroxylamine N,N-dibenzylique.

4. Composition selon les revendications 1, 2 ou 3, qui comprend en outre au moins un agent épaississant, où lesdits agents épaississants sont éventuellement choisis parmi la gomme xanthane et la gomme de guar.

5. Composition selon l'une quelconque des revendications 1 à 4, où ladite composition comprend en outre au moins un agent conservateur, où lesdits agents conservateurs sont éventuellement choisis parmi 2-méthyle-4-isothiazolin-3-one, 5-chloro-2-méthyle-4- isothiazolin-3-one, 1,2-benzisophiazolin-3-one, 2,2'-méthylènebis(5-chlorophénol), 2- bromo-2-nitropropane-1,3-diol et 1,2-octandiol.

6. Composition selon l'une quelconque revendication précédente, qui comprend en outre au moins un composé radical libre stable, où ledit au moins un composé radical libre comprend éventuellement au moins un composé nitroxyle de formule (iii) : 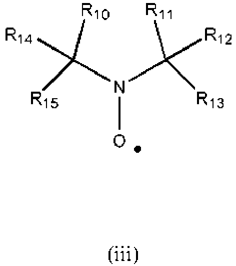 où:
R₁₀ à R₁₅ sont chacun choisis de manière indépendante parmi le groupe comprenant les hydrocarbures saturés ou insaturés, à chaîne droite ou ramifiée en C₁, C₂, C₃, C₄, C₅, C₆, C₇ ou C₈ ou les anneaux hydrocarbures saturés ou insaturés en C₅, C₆ ou C₇, et R₁₀ plus R₁₁ peuvent former ensemble un anneau saturé ou insaturé contenant un hétéro-atome supplémentaire, où l'anneau est éventuellement encore substitué par un ou plusieurs groupes alkyles ou alkényles à chaîne droite ou ramifiée ou bien une ou plusieurs fractions choisies parmi hydroxyle, oxyle, amino et alkoxyle, et où deux ou plusieurs de ces anneaux contenant du nitroxyle peuvent être joints par n'importe quel groupe de liaison.

7. Composition selon la revendication 6, où lesdits groupes de liaison englobent alkylène en C₁ à C₁₈, alkénylène en C₄ à C₁₈, xylylène, un radical acyle divalent d'un acide dicarboxylique aliphatique, araliphatique ou aromatique ou d'un acide dicarbamique ou d'un acide contenant du phosphore ou d'un acide contenant du soufre ou d'un radical silyle bivalent ou d'un sulfone, un groupe contenant du sulfure ou de l'azote ou bien un biséther, où lesdites groupes de liaison sont choisis éventuellement parmi un radical acyle d'un acide dicarboxylique aliphatique en C₂ à C₃₆, ou d'un acide cycloaliphaticque en C₈ à C₁₄ ou d'un acide dicarboxylique caromatique en C₈ à C₁₄ ou d'un acide dicarbamique aromatique en C₈ à C₁₄.

8. Composition selon la revendication 6 ou la revendication 7, où ledit composé nitroxyle est choisi parmi les composés 2,2,6,6-tétraméthyle pipéridine-1-oxyles (TEMPO) et est choisi éventuellement parmi 4-hydroxy-2,2,6,6-tétraméthylpipéridine-N-oxyle et 4-oxo-2,2,6,6- tétraméthylpipéridine-N-oxyle.

9. Composition selon l'une quelconque revendication précédente, où lesdits agents dispersants sont choisis parmi au moins tensioactif ionique ou non ionique, et éventuellement choisis parmi les esters de polyacrylate et les sels de polyarylphényléther.

10. Composition selon l'une quelconque revendication précédente, où lesdites porteurs liquides sont choisis par les liquides polaires et non polaires, et éventuellement choisis parmi l'eau, les alcools, les glycols, les cétones, les aldéhydes, les hydrocarbures aromatiques et les alcanes.

11. Composition selon l'une quelconque revendication précédente, où la teneur en contenu du composant (a) est compris entre 30:70 et 70:30 par rapport aux autres composants dans la composition.

12. Composition selon l'une quelconque revendication précédente, où la taille des particules est inférieure à 10 microns.

13. Flux de processus comprenant une composition antipolymérante en combinaison avec au moins un composé éthyléniquement insaturé, où ladite composition antipolymérante comprenant une formulation liquide concentrée, où ladite formulation liquide concentrée est un concentré de suspension qui comprend :
(a) au moins un composé choisi parmi le groupe comprenant les phénols, quinones, thiazines, hydroxylamines et amines aromatiques ;
(b) au moins un agent dispersant ; et
(c) au moins un liquide porteur polaire ou non polaire,
où ledit au moins un composé éthyléniquement insaturé comprend éventuellement un monomère aromatique vinylique ou autre monomère éthyléniquement insaturé, où ledit monomère aromatique vinylique est éventuellement du styrène ou du benzène divinylique et ledit monomère éthyléniquement insaturé est éventuellement choisi parmi l'acrylonitrile, l'acide (méth)acrylique et esters de celui-ci, le butadiène et l'isoprène, et/ou la proportion pondérale de la composition antipolymérante par rapport au composé éthyléniquement insaturé est éventuellement comprise entre 1:200 et 1:40000.

14. Procédé de prévention de réactions de polymérisation non désirées pendant la production et la transformation des composés éthyléniquement insaturés, ledit procédé comprenant le traitement d'une composition contenant au moins un composé éthyléniquement insaturé avec une composition antipolymérante, où ladite composition antipolymérante comprend une formulation liquide concentrée, où ladite formulation liquide concentrée est un concentré de suspension qui comprend :
(a) au moins un composé choisi parmi le groupe comprenant les phénols, quinones, thiazines, hydroxylamines et amines aromatiques ;
(b) au moins un agent dispersant ; et
(c) au moins un liquide porteur polaire ou non polaire,
où, éventuellement, au moins un antipolymérant est préparé sous forme de concentré de suspension et ledit concentré de suspension est injecté dans un flux de procédé.

15. Utilisation de la composition antipolymérante pour la prévention des réactions de polymérisation non désirées pendant la production et la transformation des composés éthyléniquement insaturés, où ladite composition antipolymérante comprend une formulation liquide concentrée, où ladite formulation liquide concentrée est un concentré de suspension qui comprend :
(a) au moins un composé choisi parmi le groupe comprenant les phénols, quinones, thiazines, hydroxylamines et amines aromatiques ;
(b) au moins un agent dispersant ; et
(c) au moins un liquide porteur polaire ou non polaire,
